# EUROPEAN PATENT APPLICATION

(11) **EP 4 040 153 A1**
(43) Date of publication of application: **10.08.2022**
(21) Application number: 20873277.6
(22) Date of filing: 02.09.2020
(51) Int. Cl.: G01N 33/53

(54) **METHOD FOR DETECTING TAU PROTEIN USING BLOOD SAMPLE AS SPECIMEN**

(30) Priority: 30.09.2019 JP 2019179385
(71) Applicant: Nipro Corporation, Osaka-shi, Osaka 531-8510 (JP)
(72) Inventor: KOSOKU, Yoshinori, Osaka-shi Osaka 531-8510 (JP); SANO, Yuka, Osaka-shi Osaka 531-8510 (JP); BABA, Toshiaki, Osaka-shi Osaka 531-8510 (JP); YOSHIDA, Hiroshi, Osaka-shi Osaka 531-8510 (JP)
(74) Representative: Dantz, Jan Henning
(86) International application number: PCT/JP2020/033211
(87) International publication number: WO 2021/065306

(57) **Abstract**

Two different antibodies that specifically bind to tau protein or phosphorylated tau protein are used. One of the two different antibodies is a first antibody immobilized on a support or labeled with a molecule capable of binding to the support, and the other of the two different antibodies is a second antibody labeled without being immobilized on the support. The tau protein includes an N-terminal domain, a C-terminal domain, and an intermediate domain located between the N-terminal and C-terminal domains. Epitopes recognized by the first and second antibodies are each an amino acid sequence contained in the intermediate domain or an amino acid sequence contained in the N-terminal domain. One of the first and second antibodies is first subjected to an antigen-antibody reaction with the blood sample, and the other of the first and second antibodies is subsequently subjected to an antigen-antibody reaction with the blood sample.

## Description

### Technical Field

The present invention relates to tau protein detection methods using blood samples as test specimens and particularly relates to a tau protein detection method capable of accurately detecting tau protein contained in exosomes of a blood sample.

### Background Art

Tau protein is typically expressed in nerve cells and known as a microtubule-associated protein (MAP) which promotes microtubule polymerization and contributes to microtubule stabilization.

Tau protein is known as a phosphorylatable protein in which amino acid residues such as serine or threonine residues of the amino acid sequence can be phosphorylated. Abnormalities in this phosphorylation are believed to be involved in neurodegenerative disorders such as Alzheimer's disease (AD), frontotemporal dementia (FTD), and Lewy body dementia. Thus, the use of tau protein and phosphorylated tau protein as biomarkers for neurodegenerative disorders has been conventionally studied.

A known example of specific methods for detecting tau protein or phosphorylated tau protein is disclosed in Patent Literature 1. Patent Literature 1 mentions tau protein, phosphorylated tau protein, and other molecules as biomarkers, and mentions the following biological samples as examples of the test specimen used for detecting the biomarkers: blood (whole blood), serum, plasma, urine, interstitial fluid, peritoneal fluid, cervical swab, tears, saliva, buccal swab, skin, brain tissue, and cerebrospinal fluid.

In Patent Literature 1, vesicles such as exosomes, microparticles, microvesicles, nanosomes, extracellular vesicles, or ectosomes are isolated from any of the biological samples as listed above or concentrated in the biological sample to detect at least one biomarker in the vesicles. In the case where the biomarker is phosphorylated tau protein, a composition containing an antibody is used to detect the phosphorylated tau protein.

Examples of biological samples readily available as test specimens include blood samples, which are mentioned also in Patent Literature 1. The tau protein content in blood was considered to be as low as several to several tens of picograms per mL, and thus high-sensitivity detection systems were traditionally studied. However, it has been recently found that part of tau protein in blood is present in exosomes. Thus, tau protein can be detected on the order of several hundreds of picograms per mL of a biological sample by using exosomes extracted from the biological sample as in Patent Literature 1.

Methods for directly detecting tau protein in a blood sample without extraction of exosomes have also been studied. For example, Patent Literature 2 discloses a method including: forming an immune complex on capture beads in the presence of non-capture beads, the immune complex being composed of phosphorylated tau protein in a biological sample, a capture antibody, and a detection antibody; and detecting a signal attributed to the immune complex. In Patent Literature 2, the epitope for the capture antibody and the epitope for the detection antibody are different, and the non-capture beads, on which the immune complex is not formed, are additionally present in an amount which is 1.5 times or more greater than the amount of the capture beads.

Patent Literature 2 alleges that the phosphorylated tau protein can be measured (detected) with a sensitivity high enough to distinguish between Alzheimer's disease (AD) patients and control patients since the immune complex is formed in the presence of the non-capture beads the amount of which is 1.5 or more times greater than the amount of the capture beads. However, the literature fails to explain in detail the reason why the presence of the non-capture beads allows for accurate detection (measurement) of the phosphorylated tau protein.

### Citation List

### Patent Literature

PTL 1: Japanese Laid-Open Patent Application Publication (Translation of PCT Application) No. 2016-535283
PTL 2: Japanese Laid-Open Patent Application Publication No. 2019-027952

### Summary of Invention

### Technical Problem

The present inventors extracted exosomes from a blood sample, disrupted the exosomes, and determined the amount of proteins contained in the exosomes by electrophoresis. As a result, it was confirmed that the tau protein content was on the order of nanograms per mL. This has led to the belief that the accuracy of tau protein detection can be improved if tau protein contained in exosomes of a blood sample can be detected with high responsiveness. However, conventional tau protein detection methods are cumbersome because they require a procedure for extraction of vesicles including exosomes.

Additionally, the procedure for extraction of exosomes from a blood sample is presumed to involve loss of part of tau protein contained in the blood sample. For example, in Patent Literature 1, where a drug, an antibody, or flow cytometry is used to isolate vesicles including exosomes from a biological sample or concentrate the vesicles in the biological sample, part of tau protein could be lost (partial loss could occur) depending on the conditions of the isolation or concentration, and the tau protein loss could affect the detection result.

The method as disclosed in Patent Literature 2, in which a blood sample such as plasma is used directly without extraction of exosomes, can avoid the occurrence of tau protein loss. However, in Patent Literature 2, non-capture beads need to be additionally used in an amount greater than that of capture beads for immune complex formation. Thus, in the tau protein detection method, the detection procedure could be cumbersome due to the use of a large amount of non-capture beads.

In Examples of Patent Literature 2, the proportion of the non-capture beads is discussed, and the results of distinguishing between AD patients and control patients are presented; however, the accuracy of tau protein detection and other issues are not discussed in any detail. Thus, it is unclear how sensitively tau protein can be detected by the method disclosed in Patent Literature 2.

The present invention has been made to solve the problems as described above, and an object of the present invention is to provide a detection method using a blood sample as a test specimen, the method being capable of detecting tau protein or phosphorylated tau protein with enhanced sensitivity while avoiding or reducing the cumbersomeness of the detection procedure.

### Solution to Problem

In order to solve the above problems, a tau protein detection method according to the present invention includes detecting tau protein or phosphorylated tau protein in a test specimen collected from a subject by antigen-antibody reactions using two different antibodies that specifically bind to the tau protein or phosphorylated tau protein, wherein the test specimen is a blood sample, one of the two different antibodies is a first antibody immobilized on a support or labeled with a molecule capable of binding to the support, the other of the two different antibodies is a second antibody labeled without being immobilized on the support, the tau protein includes an N-terminal domain, a C-terminal domain, and an intermediate domain located between the N-terminal and C-terminal domains, epitopes recognized by the first and second antibodies are each an amino acid sequence contained in the intermediate domain or an amino acid sequence contained in the N-terminal domain, one of the first and second antibodies is first subjected to an antigen-antibody reaction with the blood sample, and the other of the first and second antibodies is subsequently subjected to an antigen-antibody reaction with the blood sample.

In the method as defined above, the tau protein or phosphorylated tau protein in the test specimen is detected by sandwich immunoassay using a combination of the first antibody which is substantially solid-phase-immobilized and the second antibody labeled without being solid-phase-immobilized. Each of the first and second antibodies used is not an antibody the epitope of which is contained in the C-terminal domain of the tau protein, but an antibody the epitope of which is contained in the intermediate or N-terminal domain of the tau protein. Further, any non-solid-phase-immobilization support on which any antibody is not solid-phase-immobilized (non-capture support on which any antibody acting as a capture molecule is not solid-phase-immobilized) is not used in addition to the solid-phase-immobilization support on which the first antibody is solid-phase-immobilized (capture support on which the antibody acting as a capture molecule is solid-phase-immobilized).

Thus, tau protein or phosphorylated tau protein contained in exosomes of a blood sample can be accurately detected without any preliminary procedure such as exosome extraction. This makes it possible to effectively avoid the risk of tau protein loss in the course of exosome extraction and detect tau protein (or phosphorylated tau protein) with enhanced sensitivity. Not only is the need for any exosome extraction procedure eliminated, but also there is no need to prepare any non-solid-phase-immobilization support or adjust the proportion of the non-solid-phase-immobilization support relative to the solid-phase-immobilization support. Thus, the cumbersomeness of the tau protein detection can be effectively reduced or avoided. Additionally, since the first and second antibodies used may be any antibodies other than those which bind to the C-terminal domain, the flexibility in choosing the two antibodies can be increased.

In the tau protein detection method as defined above, the N-terminal domain may be composed of 1st to 44th amino acid residues of tau-441 that is one of isoforms of the tau protein, the tau-441 having an amino acid sequence that has 441 amino acid residues and that is the longest of amino acid sequences of the isoforms, and the intermediate domain may be composed of 103rd to 371st amino acid residues of the tau-441.

In the tau protein detection method as defined above, the amino acid sequence acting as the epitope recognized by the first or second antibody may be contained in a proline-rich domain included in the intermediate domain, the proline-rich domain being composed of 149th to 244th amino acid residues of the tau-441.

In the tau protein detection method as defined above, the amino acid sequence acting as the epitope recognized by the first or second antibody may be contained in a proline-rich domain included in the intermediate domain, the proline-rich domain being composed of 188th to 244th amino acid residues of the tau-441.

In the tau protein detection method as defined above, the phosphorylated tau protein may be a result of phosphorylation of at least one amino acid residue selected from 46th, 175th, 181st, 185th, 198th, 199th, 202nd, 205th, 208th, 210th, 212nd, 214th, 217th, 231st, 235th, 237th, 238th, 262nd, and 356th amino acid residues of tau-441 that is one of isoforms of the tau protein, the tau-441 having an amino acid sequence that has 441 amino acid residues and that is the longest of amino acid sequences of the isoforms.

In the tau protein detection method as defined above, the phosphorylated tau protein acting as an antigen for the first antibody may be a result of phosphorylation of at least one amino acid residue selected from the 175th, 181st, 185th, 198th, 199th, 202nd, 205th, 208th, 210th, 212nd, 214th, 217th, 231st, 235th, 237th, and 238th amino acid residues of the tau-441.

In the tau protein detection method as defined above, the support on which the first antibody is immobilized may be magnetic beads, resin beads, glass beads, a resin plate, a membrane, or a resin tube, and the molecule capable of binding to the support may be biotin or avidin.

In the tau protein detection method as defined above, a label of the second antibody may be an enzyme, a fluorescent dye, a fluorescent protein, or biotin.

In the tau protein detection method as defined above, the test specimen may be a blood sample obtained from a subject suspected of having a neurodegenerative disorder.

In the tau protein detection method as defined above, the neurodegenerative disorder may be at least one selected from Alzheimer's disease (AD), frontotemporal dementia (FTD), and Lewy body dementia.

The present invention may include a method of identifying a neurodegenerative disorder, the method including using the tau protein detection method as defined above to detect tau protein or phosphorylated tau protein in a blood sample.

The above and further objects, features and advantages of the present invention will be more apparent from the following detailed description of a preferred embodiment with reference to the accompanying drawings.

### Advantageous Effects of Invention

The present invention as defined above has the advantage of providing a detection method capable of detecting tau protein or phosphorylated tau protein with enhanced sensitivity using a blood sample as a test specimen while avoiding or reducing the cumbersomeness of the detection procedure.

### Brief Description of Drawings

FIG. 1 is a schematic diagram illustrating the structure of tau protein to be detected by a tau protein detection method according to the present invention.
FIG. 2 is a graph showing a comparison between tau protein detection results in Example 2 and Reference Example 1 for the tau protein detection method according to the present invention.
FIG. 3 is a graph showing a comparison between tau protein detection results in Example 3 and Reference Example 2 for the tau protein detection method according to the present invention.
FIG. 4 is a graph showing a comparison between tau protein detection results in Comparative Example 1 and Reference Example 3 for the tau protein detection method according to the present invention.
FIG. 5 is a graph showing a comparison between phosphorylated tau protein detection results in Example 8 and Reference Example 4 for the tau protein detection method according to the present invention.

### Description of Embodiments

Hereinafter, an exemplary embodiment of the present invention will be described in detail. A tau protein detection method according to the present disclosure includes detecting tau protein or phosphorylated tau protein in a test specimen collected from a subject by antigen-antibody reactions using two different antibodies that specifically bind to the tau protein or phosphorylated tau protein (the antibodies are referred to as anti-tau or anti-phosphorylated tau antibodies). The test specimen used is a blood sample, and the two different anti-tau or anti-phosphorylated tau antibodies used in combination are a first antibody immobilized on a support or labeled with a molecule capable of binding to the support (substantially solid-phase-immobilized antibody) and a second antibody labeled without being immobilized on the support (non-solid-phase-immobilized antibody). In the present disclosure, the anti-tau antibody that specifically binds to the tau protein and the anti-phosphorylated tau antibody that specifically binds to the phosphorylated tau protein will be collectively referred to as "tau antibody" for convenience of illustration.

The tau protein is defined as including an N-terminal domain, a C-terminal domain, and an intermediate domain located between the N-terminal and C-terminal domains. Epitopes recognized by the first and second antibodies are each an amino acid sequence contained in the intermediate domain or an amino acid sequence contained in the N-terminal domain. That is, the first antibody used may be a tau antibody the epitope of which is an amino acid sequence contained in the intermediate domain of the tau protein or phosphorylated tau protein or may be a tau antibody the epitope of which is an amino acid sequence contained in the N-terminal domain of the tau protein or phosphorylated tau protein. Likewise, the second antibody used may be a tau antibody the epitope of which is an amino acid sequence contained in the N-terminal domain of the tau protein or phosphorylated tau protein or may be a tau antibody the epitope of which is an amino acid sequence contained in the intermediate domain of the tau protein or phosphorylated tau protein.

In the tau protein detection method according to the present disclosure, one of the first and second tau antibodies is first subjected to an antigen-antibody reaction with the blood sample, and the other of the first and second tau antibodies is subsequently subjected to an antigen-antibody reaction with the blood sample. For example, the second antibody, which is not solid-phase-immobilized, may be first mixed with the blood sample and subjected to an antigen-antibody reaction to prepare a first reaction solution, and then the first reaction solution and the solid-phase-immobilized first antibody may be subjected to an antigen-antibody reaction. Alternatively, the solid-phase-immobilized first antibody may be first subjected to an antigen-antibody reaction with the blood sample to prepare a first reaction solution, and then the first reaction solution and the non-solid-phase-immobilized second antibody may be subjected to an antigen-antibody reaction.

### [Tau protein and phosphorylated tau protein]

The tau protein or phosphorylated tau protein to be detected in the present disclosure includes those mentioned in Patent Literature 1 or 2, those mentioned in Hasan A.M.M. Almansoub, et. al., "Tau Abnormalities and the Potential Therapy in Alzheimer's Disease", Journal of Alzheimer's Disease 67 (2019) pp. 13-33 (Reference Literature 1), or those mentioned in WO 2013/180238 (Reference Literature 2). The phosphorylated tau protein is a result of phosphorylation of part of amino acids forming the tau protein.

As described in Reference Literature 1 or 2, six isoforms are typically known about the tau protein. The MAPT gene, which is a tau protein-encoding gene, has 16 exons, and exons 1 to 13 are expressed in the central nervous system. Alternative splicing can occur in exons 2, 3, and 10, and thus six isoforms are produced as mentioned above.

Specifically, the number of repetitive sequences N in the N-terminal domain may be 0, 1, or 2 (0N to 2N) depending on whether alternative splicing of exon 2 or 3 of the MAPT gene occurs, and the number of repetitive sequences R in the microtubule-binding domain may be 3 (1R to 3R) or 4 (1R to 4R) depending on whether alternative splicing of exon 10 of the MAPT gene occurs. As described in Reference Literature 1, the six tau protein isoforms can be denoted as 0N3R (352 amino acid residues), 1N3R (381 amino acid residues), 2N3R (410 amino acid residues), 0N4R (383 amino acid residues), 1N4R (412 amino acid residues), and 2N4R (441 amino acid residues), respectively, based on the difference in the numbers of the repetitive sequences (the N and R denotations may be reversed as in Reference Literature 2). The tau protein to be detected in the present disclosure includes all of the six isoforms.

In the present disclosure, the ordinal numbers of amino acid residues are defined based on 2N4R which is an isoform produced without alternative splicing of any of exons 2, 3, and 10, i.e., tau protein 2N4R having an amino acid sequence that has 441 residues and that is the longest of the amino acid sequences of the six isoforms. In the present embodiment, the tau protein 2N4R is referred to as "tau-441" based on the number of the amino acid residues for convenience of illustration.

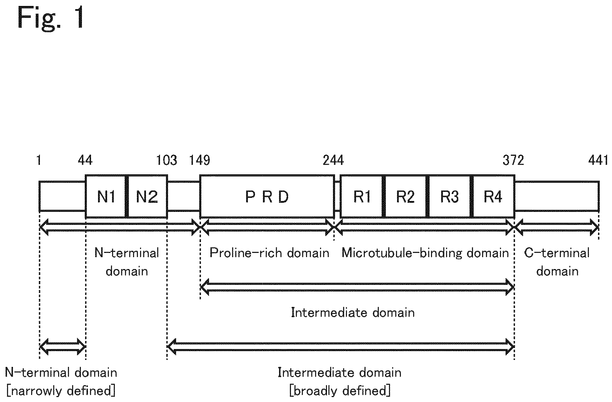

As described in Reference Literature 1 and as shown in FIG. 1, the tau-441 (2N4R) can be divided into the following four domains: an N-terminal domain, a proline-rich domain (PRD), a microtubule-binding domain (MBD), and a C-terminal domain. The N-terminal domain is composed of the 1st to 148th amino acid residues (aa 1-148), the proline-rich domain is composed of the 149th to 244th amino acid residues (aa 149-244), the microtubule-binding domain is composed of the 245th to 372nd amino acid residues (aa 244-372), and the C-terminal domain is composed of the 373rd to 441st amino acid residues (aa 373-441). In FIG. 1, the two repetitive sequences N in the N-terminal domain are denoted as "N1" and "N2", respectively, and the four repetitive sequences R in the microtubule-binding domain are denoted as "R1", "R2", "R3", and "R4", respectively.

In the present disclosure, the tau protein's amino acid sequences recognized by the tau antibodies, i.e., the amino acid sequences acting as epitopes for the tau antibodies are contained at least in the intermediate domain and, in particular, contained not only in the intermediate domain but also in the N-terminal domain. The intermediate domain of the tau protein according to the present disclosure may be any domain located between the N-terminal and C-terminal domains. Thus, a domain including the proline-rich and microtubule-binding domains, i.e., a domain composed of the 149th to 372nd amino acid residues (aa 149-372) can be defined as the intermediate domain.

In the present disclosure, the N-terminal domain (standard N-terminal domain) is defined as a wide domain extending from the N-terminal end to the proline-rich domain, and a narrow domain extending from the N-terminal end to the repetitive sequence N is designated as a "narrowly-defined" N-terminal domain. This is because the tau protein isoforms include 0N3R and 0N4R which contain no repetitive sequence N. That is, if the amino acid sequence recognized by the tau antibody is contained in the "narrowly-defined" N-terminal domain, the isoforms 0N3R and 0N4R can also be accurately detected. Specifically, the "narrowly-defined" N-terminal domain is composed of the 1st to 44th amino acid residues (aa 1-44).

When the intermediate domain is considered a domain located between the "narrowly-defined" N-terminal domain and the C-terminal domain, the intermediate domain can be broadly defined as a domain containing amino acid residues closer to the N-terminal end than the proline-rich domain. As described above, the standard N-terminal domain is composed of the 1st to 148th amino acid residues (aa 1-148), and the "narrowly-defined" N-terminal domain is composed of the 1st to 44th amino acid residues (aa 1-44). Thus, the "broadly-defined" intermediate domain can be extended to the 45th amino acid residue from the N-terminal end.

As described above, the repetitive sequences N could be absent due to alternative splicing. Thus, when the amino acid residues are numbered based on the tau-441, the intermediate domain may start from an amino acid residue next to the end of the repetitive sequences N. Since the repetitive sequences N end at the 102nd amino acid residue of the tau-441, the "broadly-defined" intermediate domain may start from the 103rd amino acid residue. Thus, in the present embodiment, the "broadly-defined" intermediate domain is preferably composed of the 103rd to 372nd amino acid residues (aa 103-372).

The "broadly-defined" intermediate domain and "narrowly-defined" N-terminal domain in the present disclosure may be defined based on the repetitive sequences N and R rather than based on the ordinal numbers of the amino acid residues of the tau-441.

Specifically, for example, the "broadly-defined" intermediate domain may start from the position next to the end of the repetitive sequence N, namely an amino acid sequence encoded by exon 2 or 3 of the MAPT gene. The "broadly-defined" intermediate domain may start from the position from which amino acid sequences encoded by exons 2 and 3 have been deleted due to alternative splicing of these exons. The "broadly-defined" intermediate domain may end at the end of the repetitive sequences R, namely amino acid sequences encoded by exons 9 to 12 of the MAPT gene. Likewise, the "narrowly-defined" N-terminal domain can be paraphrased as a domain extending from the N-terminal end to the start of the intermediate domain or a domain extending from the N-terminal end to the start of the amino acid sequence encoded by exon 2 or 3 of the MAPT gene.

The phosphorylated tau protein in the present disclosure may be a result of phosphorylation of at least one amino acid residue of the tau protein including the six isoforms described above. Typical examples of the phosphorylated tau protein include those resulting from phosphorylation of at least one amino acid residue selected from the 46th, 175th, 181st, 185th, 198th, 199th, 202nd, 205th, 208th, 210th, 212nd, 214th, 217th, 231st, 235th, 237th, 238th, 262nd, and 356th amino acid residues of the tau-441. The amino acid residues are serine or threonine residues.

More typical examples of the phosphorylated tau protein are those resulting from phosphorylation of at least one amino acid residue selected from the 175th, 181st, 185th, 198th, 199th, 202nd, 205th, 208th, 210th, 212nd, 214th, 217th, 231st, 235th, 237th, and 238th amino acid residues. These amino acid residues are contained in the proline-rich domain (aa 149-244). Thus, an anti-phosphorylated tau antibody that can recognize phosphorylation of any of these amino acid residues corresponds to the first antibody that recognizes the intermediate domain. In Examples described later, an antibody that recognizes phosphorylation of the 181st amino acid residue (threonine residue) phosphorylated is used as an example of the anti-phosphorylated tau antibody (first antibody).

The tau protein and phosphorylated tau protein in the present disclosure are not limited to the six isoforms described above, and may include any isoform (e.g., unknown isoform) different from the six isoforms. In other words, the tau protein or phosphorylated tau protein to be detected in the present disclosure is not limited to known isoforms, and may be any gene product resulting from transcription and translation from the MAPT gene (and optionally post-translational modification).

### [Tau antibodies]

A tau antibody used in the tau protein detection method according to the present disclosure is an antibody that recognizes the above tau protein or phosphorylated tau protein as an antigen. In the present disclosure, as described above, two different tau antibodies are used in combination, and the two different antibodies are a first antibody immobilized on a support or labeled with a molecule capable of binding to the support and a second antibody labeled without being immobilized on the support.

The support on which the first antibody is solid-phase-immobilized is not limited to a particular support, and a support known in the field of antigen-antibody reactions can be suitably used. Specific examples of the support include magnetic beads, resin beads, glass beads, a resin plate, a membrane, and a resin tube. The resin material (polymer compound) used in the resin beads, resin plate, or resin tube is not limited to a particular type, and a resin material known in the field of antigen-antibody reactions can be suitably used.

The magnetic beads are not limited in terms of their specific structure, and examples of the magnetic beads include magnetic particles coated with a resin material. The material used in the membrane is not limited to a particular type, and a known material such as nitrocellulose or polyvinylidene fluoride (PVDF) can be suitably used. The beads, plate, membrane, and tube are not limited in terms of their specific structure (shape and size), and may have a known structure considered suitable for the tau protein detection method.

The method for immobilizing the first antibody on the support (solid phase immobilization) is not limited to a particular technique, and a method known in the field of antigen-antibody reactions can be suitably used. Alternatively, a commercially-available antibody solid-phase immobilization kit may be used for solid-phase immobilization of the first antibody on a suitable support.

The first antibody may be labeled with a molecule capable of binding to the support instead of being directly immobilized on the support. For example, the first antibody may be labeled with a label molecule such as biotin or avidin, and the label molecule may bind to the support to accomplish solid-phase immobilization of the first antibody on the support. Thus, the first antibody in the present embodiment is not limited to an antibody solid-phase-immobilized directly on the support, and may include an antibody solid-phase-immobilized indirectly via a molecule capable of binding to the support. As such, in the present embodiment, the first antibody may be referred to as an "antibody substantially solid-phase-immobilized on the support". The label molecule capable of binding to the support is not limited to biotin or avidin mentioned above, and any of various known molecules can be used.

The label attached to the second antibody is not limited to a particular substance, and a label known in the field of antigen-antibody reactions can be suitably used. Specific examples of the label include an enzyme, a fluorescent dye, a fluorescent protein, and biotin. The method for labeling the second antibody is not limited to a particular technique, and a method known in the field of antigen-antibody reactions can be suitably used. Alternatively, a commercially-available antibody labeling kit may be used to label the second antibody with a suitable type of label.

In the present disclosure, epitopes recognized by the first and second antibodies are each an amino acid sequence contained in the intermediate domain of the tau protein or phosphorylated tau protein or an amino acid sequence contained in the N-terminal domain of the tau protein or phosphorylated tau protein. That is, the first and second antibodies may be any tau antibodies other than those the epitope of which is an amino acid sequence contained in the C-terminal domain of the tau protein or phosphorylated tau protein.

Thus, the first antibody used may be a tau antibody the epitope of which is an amino acid sequence contained in the intermediate domain of the tau protein or phosphorylated tau protein or may be a tau antibody the epitope of which is an amino acid sequence contained in the N-terminal domain of the tau protein or phosphorylated tau protein. Likewise, the second antibody used may be a tau antibody the epitope of which is an amino acid sequence contained in the N-terminal domain of the tau protein or phosphorylated tau protein or may be a tau antibody the epitope of which is an amino acid sequence contained in the intermediate domain of the tau protein or phosphorylated tau protein.

In Examples described later, the first antibody used is (1) an antibody the epitope of which is an amino acid sequence contained in the intermediate domain of the tau protein or phosphorylated tau protein, and the second antibody used is (2) an antibody the epitope of which is an amino acid sequence contained in the N-terminal domain of the tau protein or phosphorylated tau protein or (3) an antibody the epitope of which is an amino acid sequence contained in the intermediate domain of the tau protein or phosphorylated tau protein. It should be appreciated that the detection method of the present disclosure is not limited to the combination of the tau antibodies (1) and (2) or the combination of the tau antibodies (1) and (3). For example, the first antibody may be a tau antibody the epitope of which is an amino acid sequence contained in the N-terminal domain.

The method for preparing (producing) the first and second antibodies, i.e., the method for producing an antibody the epitope of which is an amino acid sequence contained in the intermediate domain of the tau protein or phosphorylated tau protein and an antibody the epitope of which is an amino acid sequence contained in the N-terminal domain of the tau protein or phosphorylated tau protein, is not limited to a particular technique, and the antibodies may be prepared by any known method using the tau protein or phosphorylated tau protein acting as an antigen.

For convenience of illustration, a tau antibody the epitope of which is an amino acid sequence contained in the intermediate domain may be referred to as "intermediate domain-recognizing tau antibody", and a tau antibody the epitope of which is an amino acid sequence contained in the N-terminal domain may be referred to as "N-terminal domain-recognizing tau antibody". In the present disclosure, a tau antibody the epitope of which is an amino acid sequence contained in the C-terminal domain and which is not used as the first or second antibody may be referred to as "C-terminal domain-recognizing tau antibody" for convenience of illustration.

The tau protein or phosphorylated tau protein acting as an antigen may be a commercially-available protein or may be prepared by a known method using genetic engineering technology. The host cells in which the tau protein or phosphorylated tau protein is expressed may be cells of a bacterium such as *Escherichia coli* (prokaryotic cells) or eukaryotic cells such as cultured cells of a yeast, an animal, or a plant. Alternatively, a cell-free protein synthesis system may be used. In Examples described later, the tau protein or phosphorylated tau protein is prepared using a yeast as a host and according to a method as described in Tom Vandebroek et al., "Identification and Isolation of a Hyperphosphorylated, Conformationally Changed Intermediate of Human Protein Tau Expressed in Yeast", BIOCHEMISTRY, Volume 44, 2005, pp. 11466-11475 (Reference Literature 3).

Examples of known methods for preparing tau antibodies (intermediate domain-recognizing tau antibody and N-terminal domain-recognizing tau antibody) using antigens include a method as described in Saeed Zarei et al., "Production and Characterization of a Peptide-based Monoclonal Antibody Against CD 44 Variant 6", MONOCLONAL ANTIBODIES IN IMMUNODIAGNOSIS AND IMMUNOTHERAPY, Volume 34, 2015, pp. 36-42 (Reference Literature 4). In Examples described later, seven different tau antibodies (including C-terminal domain-recognizing tau antibodies used in Comparative Examples) are prepared according to the method as described in Reference Literature 4.

In the method as described in Reference Literature 4, mouse spleen hybridomas are prepared by immunizing a mouse with a bound protein composed of tau protein or phosphorylated tau protein that acts as an antigen and is covalently bound to KLH (Keyhole Limpet Hemocyanin), then the hybridomas are cultured, and tau antibodies are extracted from the supernatant of the culture fluid. The recognition site of each of the tau antibodies obtained can be identified using a peptide having the corresponding amino acid sequence.

Commercially-available tau antibodies are also known. Thus, in the tau protein detection method according to the present disclosure, commercially-available antibodies may be used as the intermediate domain-recognizing tau antibody and the N-terminal domain-recognizing tau antibody if both of such antibodies are commercially available. If one of the tau antibodies is commercially available but the other tau antibody is not commercially available (e.g., if the intermediate domain-recognizing tau antibody is commercially-available but the N-terminal domain-recognizing tau antibody is not commercially available or if the intermediate domain-recognizing tau antibody used as the first antibody is commercially available but the intermediate domain-recognizing tau antibody used as the second antibody is not commercially available), the commercially-available antibody and a tau antibody prepared as described above may be used in combination.

The intermediate domain-recognizing tau antibody used as the first or second antibody in the present disclosure is a tau antibody the epitope (recognition sequence) of which is an amino acid sequence contained in the intermediate domain of the tau protein or phosphorylated tau protein. Thus, the epitope of the intermediate domain-recognizing tau antibody may be an amino acid sequence contained in the "broadly-defined" intermediate domain composed of the 45th to 372nd amino acid residues (aa 45-372) of the tau-441 or may be an amino acid sequence contained in the standard intermediate domain (domain consisting of the proline-rich and microtubule-binding domains) composed of the 149th to 372nd amino acid residues (aa 149-372) of the tau-441.

When the intermediate domain-recognizing tau antibody is an anti-phosphorylated tau antibody which recognizes phosphorylated tau protein, the phosphorylated site (the position of the phosphorylated amino acid residue) of the tau protein that is recognized by the intermediate domain-recognizing tau antibody may, as described above, be contained in the "broadly-defined" intermediate domain or the standard intermediate domain. The phosphorylated site recognized by the intermediate domain-recognizing tau antibody is preferably an amino acid sequence contained in the proline-rich domain (composed of 149th to 244th amino acid residues; aa 149-244).

The N-terminal domain-recognizing tau antibody used as the first or second antibody in the present disclosure is a tau antibody the epitope of which is an amino acid sequence contained in the N-terminal domain of the tau protein. Thus, the epitope of the N-terminal domain-recognizing tau antibody may be an amino acid sequence contained in the standard N-terminal domain composed of the 1st to 148th amino acid residues (aa 1-148) or may be an amino acid sequence contained in the "narrowly-defined" N-terminal domain composed of the 1st to 44th amino acid residues (aa 1-44).

In the tau protein detection method according to the present disclosure, there are regions of the intermediate and N-terminal domains that are more preferred as the epitopes of the intermediate domain-recognizing tau antibody and N-terminal domain-recognizing tau antibody (the regions may be referred to as "suitable regions"). Specifically, the amino acid sequence acting as the epitope of the intermediate domain-recognizing tau antibody is preferably contained in the region of the intermediate domain from the 181st to 191st amino acid residues (aa 181-191) or from the 218th to 225th amino acid residues (aa 218-225). The amino acid sequence acting as the epitope of the N-terminal domain-recognizing tau antibody is preferably contained in the region of the N-terminal domain from the 1st to 20th amino acid residues (aa 1-20) or from the 16th to 24th amino acid residues (aa 16-24). When the epitopes of the intermediate domain-recognizing tau antibody and N-terminal domain-recognizing tau antibody are amino acid sequences contained in the suitable regions, the accuracy of detection of the tau protein or phosphorylated tau protein can be further improved.

### [Tau protein detection method]

In the tau protein detection method according to the present disclosure, a blood sample is used as a test specimen, and the two different antibodies, i.e., the first and second antibodies described above, are reacted with the test specimen to detect the tau protein or phosphorylated tau protein in the test specimen. In the present disclosure, as described above, the first and second antibodies used are not tau antibodies the epitopes of which are contained in the C-terminal domain of the tau protein or phosphorylated tau protein, but tau antibodies the epitopes of which are contained in the intermediate domain or N-terminal domain of the tau protein or phosphorylated tau protein. Further, any non-solid-phase-immobilization support on which any antibody is not solid-phase-immobilized (non-capture support on which any antibody acting as a capture molecule is not solid-phase-immobilized) is not used in addition to the solid-phase-immobilization support on which the first antibody is solid-phase-immobilized (capture support on which the antibody acting as a capture molecule is solid-phase-immobilized). This leads to accurate detection of the tau protein or phosphorylated tau protein contained in exosomes of the blood sample.

In Examples of Patent Literature 2, a commercially-available antibody that recognizes an amino acid sequence composed of the 159th to 163rd residues (aa 159-163) of tau protein is used as a "capture antibody", and a commercially-available antibody that recognizes phosphorylation of the 181st amino acid residue (threonine residue) of phosphorylated tau protein is used as a "detection antibody". The capture antibody is conjugated to capture beads, and this capture antibody is bound to the target protein (phosphorylated tau protein) first. The detection antibody is then bound to the target protein binding to the capture antibody, thus forming a capture antibody-target protein-detection antibody immune complex.

The capture antibody conjugated to the capture beads recognizes the amino acid sequence aa 159-163 of tau protein; thus, this capture antibody corresponds to the first antibody and intermediate domain-recognizing tau antibody in the present disclosure. The detection antibody is an antibody labeled with biotin without being conjugated to the capture beads and recognizes phosphorylation of the 181st amino acid residue of phosphorylated tau protein; thus, this detection antibody corresponds to the second antibody and intermediate domain-recognizing tau antibody in the present disclosure.

In both Examples and embodiments of Patent Literature 2, the capture antibody corresponding to the first antibody in the present disclosure is first reacted with the phosphorylated tau protein, and then the detection antibody corresponding to the second antibody is reacted with the phosphorylated tau protein. Although details are unclear, the reason for the need of a large amount of non-capture beads (non-solid-phase-immobilization support on which any antibody is not solid-phase-immobilized) in Patent Literature 2 may be that the two different antibodies are reacted with the tau protein in the order as described above.

In Examples of Patent Literature 2, the results of distinguishing between AD patients and control patients are presented, but the accuracy of tau protein detection and other issues are not discussed in any detail. Examples of Patent Literature 2 demonstrate that a decreased amount of non-capture beads results in a failure to fully distinguish AD patients. This implies that the accuracy of phosphorylated tau protein detection by the method of Patent Literature 2 is insufficient to fully detect tau protein contained in exosomes.

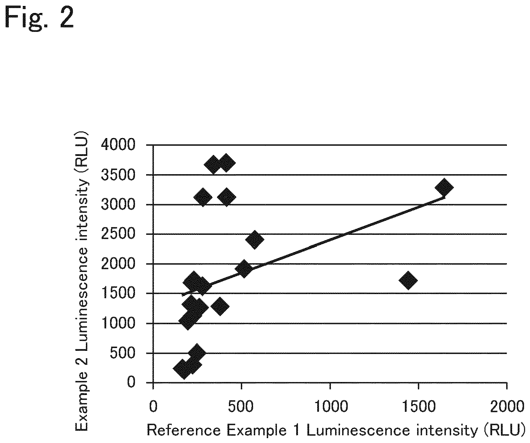

In the tau protein detection method according to the present disclosure, as is evident from comparisons between the results of Examples and Reference Examples described later, there is a significant correlation between the result of detection of the tau protein or phosphorylated tau protein in exosomes extracted from a blood sample and the result of detection of the tau protein or phosphorylated tau protein in the blood sample itself (FIGS. 2 to 5). Additionally, as is evident from the results of Examples and Comparative Examples, the tau protein detection method according to the present disclosure offers such a high detection accuracy that the sensitivity is 70% or more, the specificity is 70% or more, and the area under ROC curve (Receiver Operating Characteristic curve) is 0.70 or more.

It is therefore concluded that the tau protein detection method according to the present disclosure is capable of detecting the tau protein or phosphorylated tau protein with high accuracy both because any tau antibody the epitope of which is contained in the C-terminal domain is not used and because the solid-phase-immobilization support on which the first antibody is solid-phase-immobilized is used alone without additionally using any non-solid phase-immobilization support (non-capture support) on which any antibody is not solid-phase-immobilized.

The tau protein detection method according to the present disclosure can detect the tau protein or phosphorylated tau protein contained in exosomes without extracting the exosomes from a blood sample used as a test specimen. This makes it possible to effectively avoid the risk of tau protein loss in the course of exosome extraction and detect the tau protein (or phosphorylated tau protein) with enhanced sensitivity. Additionally, the cumbersomeness of the tau protein detection can be effectively reduced or avoided.

As described above, the test specimen used in the tau protein detection method according to the present disclosure may be any blood sample and is not limited to a particular blood component. Specific examples of the blood sample include whole blood, plasma, and serum. Among these, plasma is more preferred. The method for separating plasma from whole blood is not limited to a particular technique, and the plasma used may be one separated from whole blood collected by a known blood drawing method using an anticoagulant such as a heparin salt, a citric salt, or an ethylenediaminetetraacetic (EDTA) salt.

As described above, the tau protein detection method according to the present disclosure requires that: tau antibodies adapted to recognize specific recognition sites (epitopes) in the tau protein or phosphorylated tau protein be used in combination; one of the tau antibodies be the first antibody substantially solid-phase-immobilized on a support, and the other tau antibody be the labeled second antibody; and the first and second antibodies be individually reacted with a blood sample used as a test specimen. The tau protein detection method is not limited in any other respects, such as in terms of other specific procedures and the conditions and order of the other procedures.

In Examples described later, the blood sample used as the test specimen is diluted as appropriate and reacted first with the second antibody and then with the first antibody, and the reactions are followed by separation of the support and washing of the immune complex. These procedures are merely an example, and the tau protein detection method according to the present disclosure is not limited in terms of the specific methods, conditions, and reagents used for the order of the reactions of the first and second antibodies, the dilution of the blood sample, the separation of the support, and the washing of the immune complex. As long as the tau protein or phosphorylated tau protein can be accurately detected, the order of the reactions of the first and second antibodies may be chosen as appropriate, part of the procedures including the dilution, separation, and washing may be skipped, another procedure may be carried out, the order of the procedures may be changed, the reagent used in a procedure may be changed, or the same procedure may be repeated a plurality of times.

The specific method for detection of the tau protein or phosphorylated tau protein is not limited to a particular technique. In Examples described later, a luminescent substrate is added and reacted on the washed support, and the amount of luminescence (luminescence intensity) is measured with a commercially-available photometer to detect the tau protein or phosphorylated tau protein. However, the detection of the tau protein or phosphorylated tau protein is not limited to being performed by the method based on measurement of the amount of luminescence, and another known method may be used. For example, the tau protein or phosphorylated tau protein may be detected by using a known detection kit.

The tau protein detection method according to the present disclosure is capable of accurately detecting tau protein or phosphorylated tau protein and thus suitable for use in identification (distinguishing or diagnosis) of a neurodegenerative disorder. Hence, the scope of the present disclosure embraces a method of identifying a neurodegenerative disorder of a subject such as a human or mammal from which a blood sample has been collected as a test specimen, the method including using the tau protein detection method described above to detect tau protein or phosphorylated tau protein in the blood sample.

Thus, the test specimen used in the tau protein detection method according to the present disclosure may be a blood sample obtained from a subject suspected of having a neurodegenerative disorder. The subject is not limited to a patient diagnosed with a neurodegenerative disorder or presenting suspected symptoms of the disorder, and may be a healthy subject suffering from no symptoms but wanting to confirm the possibility of any neurodegenerative disorder.

The neurodegenerative disorder to be identified using the tau protein detection method according to the present disclosure may be any disorder that is caused (or that can be caused or is considered to be caused) in part by tau protein-related abnormalities. Disorders believed to be attributable primarily to abnormal accumulation of phosphorylated tau protein are collectively referred to as "tauopathies". The tau protein detection method according to the present disclosure is suitable for use in identification of such tauopathies. Specific examples of the neurodegenerative disorder include, but are not limited to, Alzheimer's disease (AD), frontotemporal dementia (FTD), and Lewy body dementia.

### Examples

The present invention will be described in more detail based on Examples, Comparative Examples, and Reference Examples. The present invention is not limited to Examples given below. Skilled persons in the art can make various changes, modifications, or alterations without departing from the scope of the present invention. The antibodies, test specimens, reagents etc., and detection method used in Examples, Comparative Examples, and Reference Examples were as described below.

Antibodies, test specimens, reagents etc., and detection method

### [Tau antibodies]

The tau antibodies used in Examples, Comparative Examples, and Reference Examples are listed in Table 1. The tau antibodies were prepared according to the method of Reference Literature 4 mentioned above. As previously stated, the tau protein or phosphorylated tau protein acting as an antigen was prepared according to the method of Reference Literature 3. The recognition site of each tau antibody was identified using a peptide having the corresponding amino acid sequence.

**[Table 1]**

| Symbol for antibody | Recognition site | Classification |
|---|---|---|
| T1 | aa 1-20 [N-terminal domain] | N-terminal domain-recognizing antibody |
| T2 | aa 16-24 [N-terminal domain] | N-terminal domain-recognizing antibody |
| T3 | aa 185-191 [Intermediate domain] | Intermediate domain-recognizing antibody |
| T4 | aa 218-225 [Intermediate domain] | Intermediate domain-recognizing antibody |
| T5 | aa 395-417 [C-terminal domain] | C-terminal domain-recognizing antibody |
| T6 | aa 428-441 [C-terminal domain] | C-terminal domain-recognizing antibody |
| Tp | pT 181 [Phosphorylation of intermediate domain] | Intermediate domain-recognizing antibody |

### [Test specimens]

A total of 20 plasma samples were used as test specimens in Examples and Comparative Examples. Ten of the plasma samples were donated from 10 AD patients and the other ten plasma samples were donated from 10 healthy subjects. In Reference Examples, 20 extracts (exosome extracts) obtained by extracting exosomes from the 20 plasma samples through polymer precipitation were used.

### [Reagents etc.]

A phosphate buffer solution containing 2.0% bovine serum albumin was used as a diluent for diluting the test specimens. A phosphate buffer solution containing 0.05% Tween 20 was used as a cleaning fluid. Lumigen APS-5 manufactured by FU-JIFILM Wako Pure Chemical Corporation was used as a luminescent substrate.

Dynabeads (magnetic beads) manufactured by Thermo Fisher Scientific were used as a support on which the first antibody was solid-phase-immobilized. The first antibody was solid-phase-immobilized on the magnetic beads using a method described in the instructions attached to Dynabeads.

Purified calf-intestinal alkaline phosphatase (ALP) manufactured by Thermo Fisher Scientific was used as a label of the second antibody. The second antibody was labeled with the ALP by using Alkaline Phosphatase Labeling Kit of Dojindo Laboratories according to a method described in the attached instructions.

The amount of luminescence was measured using CL-JACK NX, a photometer manufactured by Hitachi Chemical Diagnostic Systems.

### [Tau protein detection method]

One part by volume of human plasma (Example or Comparative Example) or exosome extract (Reference Example) obtained as the test specimen was mixed with one part by volume of the diluent to prepare a diluted test specimen. Subsequently, two parts by volume of the diluent and four parts by volume of an ALP-labeled antibody solution containing the second antibody were added and mixed with two parts by volume of the diluted test specimen. A first reaction solution was thus prepared, and the reaction was allowed to proceed at 37□C for a given period of time.

After the reaction, four parts by volume of the magnetic beads with the immobilized first antibody were added and mixed with eight parts by volume of the first reaction solution. A second reaction solution was thus prepared, and the reaction was allowed to proceed at 37□C for a given period of time.

After the reaction, the magnetic beads were separated from the second reaction solution and washed with the cleaning fluid. To the washed magnetic beads were added eight parts by volume of the luminescent substrate, and the reaction was allowed to proceed at 25□C for several to less than 20 seconds. The luminescence intensity (amount of luminescence) was measured to detect tau protein or phosphorylated tau protein.

In each of Examples, Comparative Examples, and Reference Examples, the results of tau protein or phosphorylated tau protein detection in the 20 test specimens were used as a basis to calculate the sensitivity and specificity, create an ROC curve, and calculate the area under the ROC curve (ROC area). In Examples and Comparative Examples, it was determined that a sufficiently high detection accuracy was achieved in the case where the sensitivity was 70% or more, the specificity was 70% or more, and the ROC area was 0.70 or more. Reference Examples, where the exosome extracts were used, are experiments conducted for comparisons with Examples or Comparative Examples. Thus, for Reference Examples, graphs showing the comparisons are presented in the drawings, and the results of the parameters such as the sensitivity are omitted.

### Example 1

Antibody T4, i.e., an intermediate domain-recognizing tau antibody that recognizes an amino acid sequence composed of the 218th to 225th residues (aa 218-225) of tau-441, was used as the first antibody, and antibody T1, i.e., an N-terminal domain-recognizing tau antibody that recognizes an amino acid sequence composed of the 1st to 20th residues (aa 1-20) of tau-441, was used as the second antibody. For the 20 test specimens (plasma samples), the amount of luminescence was measured as described above to detect tau protein.

The combination of the first and second antibodies in Example 1 was evaluated for the sensitivity, specificity, and ROC area based on the result of tau protein detection. The evaluation results are listed in Table 2.

### Example 2

For the 20 test specimens (plasma samples), the amount of luminescence was measured to detect tau protein in the same manner as in Example 1, except that the second antibody used was antibody T2, i.e., an N-terminal domain-recognizing tau antibody that recognizes an amino acid sequence composed of the 16th to 24th residues of tau-441. The combination of the first and second antibodies in Example 2 was evaluated for the sensitivity, specificity, and ROC area based on the result of tau protein detection. The evaluation results are listed in Table 2.

The detection result (luminescence intensity) in Example 2 was plotted on the ordinate and the detection result (luminescence intensity) in Reference Example 1 described later was plotted on the abscissa to compare these detection results. The resulting graph is shown in FIG. 2.

### Example 3

For the 20 test specimens (plasma samples), the amount of luminescence was measured to detect tau protein in the same manner as in Example 1, except that the second antibody used was antibody T3, i.e., an intermediate domain-recognizing tau antibody that recognizes an amino acid sequence composed of the 185th to 191st residues of tau-441. The combination of the first and second antibodies in Example 3 was evaluated for the sensitivity, specificity, and ROC area based on the result of tau protein detection. The evaluation results are listed in Table 2.

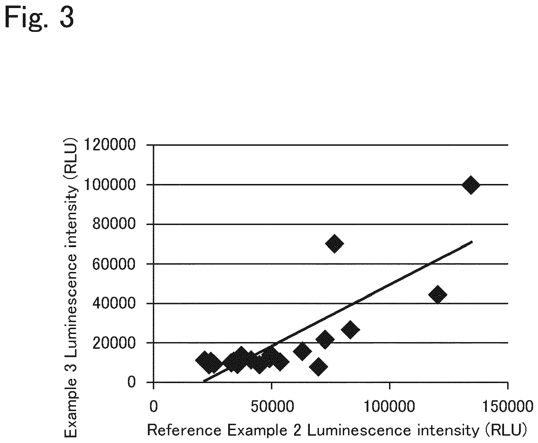

The detection result (luminescence intensity) in Example 3 was plotted on the ordinate and the detection result (luminescence intensity) in Reference Example 2 described later was plotted on the abscissa to compare these detection results. The resulting graph is shown in FIG. 3.

### Example 4

For the 20 test specimens (plasma samples), the amount of luminescence was measured to detect tau protein in the same manner as in Example 1, except that the second antibody used was antibody T4, i.e., an intermediate domain-recognizing tau antibody identical to the first antibody. The combination of the first and second antibodies in Example 4 was evaluated for the sensitivity, specificity, and ROC area based on the result of tau protein detection. The evaluation results are listed in Table 2.

### Comparative Example 1

For the 20 test specimens (plasma samples), the amount of luminescence was measured to detect tau protein in the same manner as in Example 1, except that the second antibody used was antibody T5, i.e., a comparative antibody that recognizes an amino acid sequence composed of the 395th to 417th residues of tau-441. The combination of the first and second antibodies in Comparative Example 1 was evaluated for the sensitivity, specificity, and ROC area based on the result of tau protein detection. The evaluation results are listed in Table 2.

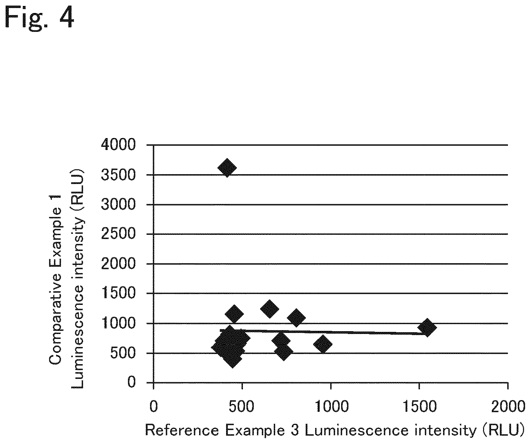

The detection result (luminescence intensity) in Comparative Example 1 was plotted on the ordinate and the detection result (luminescence intensity) in Reference Example 3 described later was plotted on the abscissa to compare these detection results. The resulting graph is shown in FIG. 4.

### Comparative Example 2

For the 20 test specimens (plasma samples), the amount of luminescence was measured to detect tau protein in the same manner as in Example 1, except that the second antibody used was antibody T6, i.e., a comparative antibody that recognizes an amino acid sequence composed of the 428th to 441st residues of tau-441. The combination of the first and second antibodies in Comparative Example 2 was evaluated for the sensitivity, specificity, and ROC area based on the result of tau protein detection. The evaluation results are listed in Table 2.

### Reference Example 1

For the 20 test specimens (plasma samples), the amount of luminescence was measured to detect tau protein in the same manner as in Example 2, except that the exosome extracts as described above were used as the test specimens instead of the plasma samples.

The detection result (luminescence intensity) in Example 2 was plotted on the ordinate and the detection result (luminescence intensity) in Reference Example 1 was plotted on the abscissa to compare these detection results. The resulting graph is shown in FIG. 2.

### Reference Example 2

For the 20 test specimens (plasma samples), the amount of luminescence was measured to detect tau protein in the same manner as in Example 3, except that the exosome extracts as described above were used as the test specimens instead of the plasma samples.

The detection result (luminescence intensity) in Example 3 was plotted on the ordinate and the detection result (luminescence intensity) in Reference Example 2 was plotted on the abscissa to compare these detection results. The resulting graph is shown in FIG. 3.

### Reference Example 3

For the 20 test specimens (plasma samples), the amount of luminescence was measured to detect tau protein in the same manner as in Comparative Example 1, except that the exosome extracts as described above were used as the test specimens instead of the plasma samples.

The detection result (luminescence intensity) in Comparative Example 1 was plotted on the ordinate and the detection result (luminescence intensity) in Reference Example 3 was plotted on the abscissa to compare these detection results. The resulting graph is shown in FIG. 4.

**[Table 2]**

| | | Example 1 | Example 2 | Example 3 | Example 4 | Compara-tive Example 1 | Comparative Example 2 |
|---|---|---|---|---|---|---|---|
| Antibody | First | T4 | T4 | T4 | T4 | T4 | T4 |
| | Second | T1 | T2 | T3 | T4 | T5 | T6 |
| Result | Sensitivity | 70% | 70% | 70% | 80% | 60% | 50% |
| | Specificity | 70% | 70% | 80% | 70% | 60% | 70% |
| | ROC area | 0.76 | 0.75 | 0.74 | 0.80 | 0.54 | 0.66 |

### Example 5

Antibody Tp, i.e., an intermediate domain-recognizing tau antibody against phosphorylated tau protein (pT 181) resulting from phosphorylation of the 181st amino acid residue (threonine residue) of tau-441 (antibody that recognizes a phosphorylated site in the intermediate domain), was used as the first antibody, and antibody T1, i.e., an N-terminal domain-recognizing tau antibody that recognizes an amino acid sequence composed of the 1st to 20th residues of tau-441, was used as the second antibody. For the 20 test specimens (plasma samples), the amount of luminescence was measured as described above to detect phosphorylated tau protein.

The combination of the first and second antibodies in Example 5 was evaluated for the sensitivity, specificity, and ROC area based on the result of phosphorylated tau protein detection. The evaluation results are listed in Table 3.

### Example 6

For the 20 test specimens (plasma samples), the amount of luminescence was measured to detect phosphorylated tau protein in the same manner as in Example 5, except that the second antibody used was antibody T2, i.e., an N-terminal domain-recognizing tau antibody that recognizes an amino acid sequence composed of the 16th to 24th residues of tau-441. The combination of the first and second antibodies in Example 6 was evaluated for the sensitivity, specificity, and ROC area based on the result of phosphorylated tau protein detection. The evaluation results are listed in Table 3.

### Example 7

For the 20 test specimens (plasma samples), the amount of luminescence was measured to detect phosphorylated tau protein in the same manner as in Example 5, except that the second antibody used was antibody T3, i.e., an intermediate domain-recognizing tau antibody that recognizes an amino acid sequence composed of the 185th to 191st residues of tau-441. The combination of the first and second antibodies in Example 7 was evaluated for the sensitivity, specificity, and ROC area based on the result of phosphorylated tau protein detection. The evaluation results are listed in Table 3.

### Example 8

For the 20 test specimens (plasma samples), the amount of luminescence was measured to detect phosphorylated tau protein in the same manner as in Example 5, except that the second antibody used was antibody T4, i.e., an intermediate domain-recognizing tau antibody that recognizes an amino acid sequence composed of the 218th to 225th residues of tau-441. The combination of the first and second antibodies in Example 8 was evaluated for the sensitivity, specificity, and ROC area based on the result of phosphorylated tau protein detection. The evaluation results are listed in Table 3.

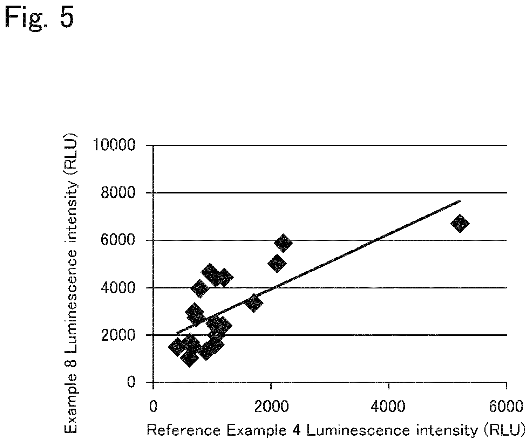

The detection result (luminescence intensity) in Example 8 was plotted on the ordinate and the detection result (luminescence intensity) in Reference Example 4 described later was plotted on the abscissa to compare these detection results. The resulting graph is shown in FIG. 5.

### Comparative Example 3

For the 20 test specimens (plasma samples), the amount of luminescence was measured to detect phosphorylated tau protein in the same manner as in Example 5, except that the second antibody used was antibody T5, i.e., a comparative antibody that recognizes an amino acid sequence composed of the 395th to 417th residues of tau-441. The combination of the first and second antibodies in Comparative Example 3 was evaluated for the sensitivity, specificity, and ROC area based on the result of phosphorylated tau protein detection. The evaluation results are listed in Table 2.

### Comparative Example 4

For the 20 test specimens (plasma samples), the amount of luminescence was measured to detect phosphorylated tau protein in the same manner as in Example 5, except that the second antibody used was antibody T6, i.e., a comparative antibody that recognizes an amino acid sequence composed of the 428th to 441st residues of tau-441. The combination of the first and second antibodies in Comparative Example 4 was evaluated for the sensitivity, specificity, and ROC area based on the result of phosphorylated tau protein detection. The evaluation results are listed in Table 2.

### Reference Example 4

For the 20 test specimens (plasma samples), the amount of luminescence was measured to detect phosphorylated tau protein in the same manner as in Example 8, except that the exosome extracts as described above were used as the test specimens instead of the plasma samples.

The detection result (luminescence intensity) in Example 8 was plotted on the ordinate and the detection result (luminescence intensity) in Reference Example 4 was plotted on the abscissa to compare these detection results. The resulting graph is shown in FIG. 5.

**[Table 3]**

| | | Example 5 | Example 6 | Example 7 | Example 8 | Comparative Example 3 | Comparative Example 4 |
|---|---|---|---|---|---|---|---|
| Antibody | First | Tp | Tp | Tp | Tp | Tp | Tp |
| | Second | T1 | T2 | T3 | T4 | T5 | T6 |
| Result | Sensitivity | 80% | 80% | 100% | 90% | 50% | 50% |
| | Specificity | 90% | 80% | 90% | 90% | 70% | 70% |
| | ROC area | 0.78 | 0.86 | 0.93 | 0.93 | 0.69 | 0.57 |

### Comparisons among Examples, Comparative Examples, and Reference Examples

As is evident from the results of Examples 1 to 4 and Comparative Examples 1 and 2, the tau protein detection method according to the present disclosure can detect tau protein with high accuracy. Likewise, as is evident from the results of Examples 5 to 8 and Comparative Examples 3 and 4, the tau protein detection method according to the present disclosure can detect phosphorylated tau protein with high accuracy.

As seen from FIGS. 2 to 5, Example 2, Example 3, Comparative Example 1, and Example 8 respectively showed significant correlations with Reference Examples 1 to 4 where the exosome extracts were used (the straight lines in the drawings). This leads to the conclusion that the detection method according to the present disclosure, which uses a blood sample such as plasma as a test specimen, properly reflects a detection result as obtained using an exosome extract. Thus, the method according to the present disclosure can accurately detect tau protein or phosphorylated tau protein while effectively avoiding the risk of tau protein loss in the course of exosome extraction.

Reference Examples using exosome extracts were conducted in one-to-one correspondence with Examples 1 to 8 and Comparative Examples 1 to 4. However, only Reference Examples corresponding to typical ones of Examples and Comparative Examples are discussed in the detailed description of the present invention, and comparisons of the typical ones of Examples and Comparative Examples with the corresponding ones of Reference Examples are shown in the drawings (Example 2 is an example using an N-terminal domain-recognizing tau antibody (T2) the epitope of which is contained in the N-terminal domain, Example 3 is an example using an intermediate domain-recognizing tau antibody (T3) the epitope of which is contained in the intermediate domain, Comparative Example 1 is an example using a comparative antibody (T5) the epitope of which is contained in the C-terminal domain, and Example 8 is an example using another intermediate domain-recognizing tau antibody (T4) the epitope of which is contained in the intermediate domain.)

All of Examples and Comparative Examples respectively showed significant correlations with the corresponding ones of Reference Examples, although not all of the results of comparisons of Examples and Comparative Examples with Reference Examples are shown for convenience of illustration. It is seen that Comparative Examples 1 to 4 are similar to Examples 1 to 8 in proper reflection of detection results as obtained using exosome extracts, although the detection accuracy was lower in Comparative Examples 1 to 4 than in Examples 1 to 8 because the second antibodies used in Comparative Examples 1 to 4 were those which recognize an amino acid sequence contained in the C-terminal domain.

As described above, the tau protein detection method according to the present disclosure includes detecting tau protein or phosphorylated tau protein in a test specimen collected from a subject by antigen-antibody reactions using two different antibodies that specifically bind to the tau protein or phosphorylated tau protein, wherein the test specimen is a blood sample, one of the two different antibodies is a first antibody immobilized on a support or labeled with a molecule capable of binding to the support, the other of the two different antibodies is a second antibody labeled without being immobilized on the support, the tau protein includes an N-terminal domain, a C-terminal domain, and an intermediate domain located between the N-terminal and C-terminal domains, epitopes recognized by the first and second antibodies are each an amino acid sequence contained in the intermediate domain or an amino acid sequence contained in the N-terminal domain, one of the first and second antibodies is first subjected to an antigen-antibody reaction with the blood sample, and the other of the first and second antibodies is subsequently subjected to an antigen-antibody reaction with the blood sample.

In the method as described above, the tau protein or phosphorylated tau protein in the test specimen is detected by sandwich immunoassay using a combination of the first antibody which is substantially solid-phase-immobilized and the second antibody labeled without being solid-phase-immobilized. Each of the first and second antibodies used is not an antibody the epitope of which is contained in the C-terminal domain of the tau protein, but an antibody the epitope of which is contained in the intermediate or N-terminal domain of the tau protein. Further, any non-solid-phase-immobilization support on which any antibody is not solid-phase-immobilized (non-capture support on which any antibody acting as a capture molecule is not solid-phase-immobilized) is not used in addition to the solid-phase-immobilization support on which the first antibody is solid-phase-immobilized (capture support on which the antibody acting as a capture molecule is solid-phase-immobilized).

Thus, tau protein or phosphorylated tau protein contained in exosomes of a blood sample can be accurately detected without any preliminary procedure such as exosome extraction. This makes it possible to effectively avoid the risk of tau protein loss in the course of exosome extraction and detect tau protein (or phosphorylated tau protein) with enhanced sensitivity. Not only is the need for any exosome extraction procedure eliminated, but also there is no need to prepare any non-solid-phase-immobilization support or adjust the proportion of the non-solid-phase-immobilization support relative to the solid-phase-immobilization support. Thus, the cumbersomeness of the tau protein detection can be effectively reduced or avoided. Additionally, since the first and second antibodies used may be any antibodies other than those which bind to the C-terminal domain, the flexibility in choosing the two different antibodies can be increased.

The present invention is not limited to the embodiment described above, and various modifications can be made without departing from the scope as defined by the appended claims. The technical scope of the present invention encompasses embodiments obtained by combining technical features disclosed in different embodiments or variants.

From the foregoing description, numerous modifications and other embodiments of the present invention are obvious to those skilled in the art. Accordingly, the foregoing description is to be construed as illustrative only, and is provided for the purpose of teaching those skilled in the art the best mode for carrying out the present invention. The structural and/or functional details may be substantially modified without departing from the scope of the present invention.

### Industrial Applicability

The present invention is suitable for wide use in fields involving detection of tau protein or phosphorylated tau protein in a test specimen and also for wide use in various application fields using tau protein or phosphorylated tau protein detection.

## Claims

1. A tau protein detection method comprising detecting tau protein or phosphorylated tau protein in a test specimen collected from a subject by antigen-antibody reactions using two different antibodies that specifically bind to the tau protein or phosphorylated tau protein, wherein
the test specimen is a blood sample,
one of the two different antibodies is a first antibody immobilized on a support or labeled with a molecule capable of binding to the support,
the other of the two different antibodies is a second antibody labeled without being immobilized on the support,
the tau protein includes an N-terminal domain, a C-terminal domain, and an intermediate domain located between the N-terminal and C-terminal domains, epitopes recognized by the first and second antibodies are each an amino acid sequence contained in the intermediate domain or an amino acid sequence contained in the N-terminal domain,
one of the first and second antibodies is first subjected to an antigen-antibody reaction with the blood sample, and
the other of the first and second antibodies is subsequently subjected to an antigen-antibody reaction with the blood sample.

2. The tau protein detection method according to claim 1, wherein
the N-terminal domain is composed of 1st to 44th amino acid residues of tau-441 that is one of isoforms of the tau protein, the tau-441 having an amino acid sequence that has 441 amino acid residues and that is the longest of amino acid sequences of the isoforms, and
the intermediate domain is composed of 103rd to 371st amino acid residues of the tau-441.

3. The tau protein detection method according to claim 2, wherein the amino acid sequence acting as the epitope recognized by the first or second antibody is contained in a proline-rich domain included in the intermediate domain, the proline-rich domain being composed of 149th to 244th amino acid residues of the tau-441.

4. The tau protein detection method according to claim 2, wherein the amino acid sequence acting as the epitope recognized by the first or second antibody is contained in a proline-rich domain included in the intermediate domain, the proline-rich domain being composed of 188th to 244th amino acid residues of the tau-441.

5. The tau protein detection method according to any one of claims 1 to 4, wherein the phosphorylated tau protein is a result of phosphorylation of at least one amino acid residue selected from 46th, 175th, 181st, 185th, 198th, 199th, 202nd, 205th, 208th, 210th, 212nd, 214th, 217th, 231st, 235th, 237th, 238th, 262nd, and 356th amino acid residues of tau-441 that is one of isoforms of the tau protein, the tau-441 having an amino acid sequence that has 441 amino acid residues and that is the longest of amino acid sequences of the isoforms.

6. The tau protein detection method according to claim 5, wherein the phosphorylated tau protein acting as an antigen for the first antibody is a result of phosphorylation of at least one amino acid residue selected from the 175th, 181st, 185th, 198th, 199th, 202nd, 205th, 208th, 210th, 212nd, 214th, 217th, 231st, 235th, 237th, and 238th amino acid residues of the tau-441.

7. The tau protein detection method according to any one of claims 1 to 6, wherein the support on which the first antibody is immobilized is magnetic beads, resin beads, glass beads, a resin plate, a membrane, or a resin tube, and
the molecule capable of binding to the support is biotin or avidin.

8. The tau protein detection method according to any one of claims 1 to 7, wherein a label of the second antibody is an enzyme, a fluorescent dye, a fluorescent protein, or biotin.

9. The tau protein detection method according to any one of claims 1 to 8, wherein the test specimen is a blood sample obtained from a subject suspected of having a neurodegenerative disorder.

10. The tau protein detection method according to claim 9, wherein the neurodegenerative disorder is at least one selected from Alzheimer's disease (AD), frontotemporal dementia (FTD), and Lewy body dementia.

11. A method of identifying a neurodegenerative disorder, the method comprising using the tau protein detection method according to any one of claims 1 to 10 to detect tau protein or phosphorylated tau protein in a blood sample.
